(19) Europäisches Patentamt
European Patent Office
Office européen des brevets

(11) Veröffentlichungsnummer: **0 332 033 A2**

(12) # EUROPÄISCHE PATENTANMELDUNG

(21) Anmeldenummer: **89103547.9**

(22) Anmeldetag: **01.03.89**

(51) Int. Cl.⁴: **C07D 215/56 , C07C 93/12**

(30) Priorität: **11.03.88 DE 3808117**

(43) Veröffentlichungstag der Anmeldung:
**13.09.89 Patentblatt 89/37**

(84) Benannte Vertragsstaaten:
**AT BE CH DE ES FR GB GR IT LI NL SE**

(71) Anmelder: **BAYER AG**

**D-5090 Leverkusen 1 Bayerwerk(DE)**

(72) Erfinder: **Schriewer, Michael, Dr.**
**Am Thelen Siefen 1 a**
**D-5068 Odenthal(DE)**
Erfinder: **Petersen, Uwe, Dr.**
**Auf dem Forst 4**
**D-5090 Leverkusen 1(DE)**
Erfinder: **Grohe, Klaus, Dr.**
**Am Wasserturm 10**
**D-5068 Odenthal(DE)**
Erfinder: **Krebs, Andreas, Dr.**
**Am Gartenfeld 70**
**D-5068 Odenthal(DE)**

(54) **N-Cyclopropylaniline und deren Verwendung in Verfahren zur Herstellung von 1-Cyclopropyl-chinoloncarbonsäuren und deren Derivaten.**

(57) Die Erfindung betrifft N-Cyclopropylaniline der Formel (IV)

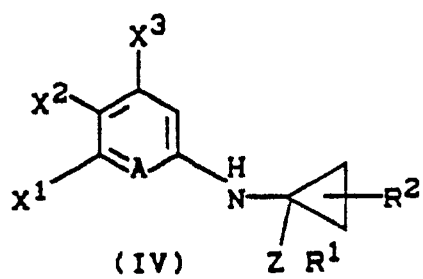

(IV)

in welcher $R^1$, $X^1$, $X^2$, $X^3$, A, Z und $R^2$ die in der Beschreibung angegebene Bedeutung haben, die sich zur Herstellung von 4-Chinolon-3-carbonsäuren eignen, sowie Verfahren zur Herstellung von Chinoloncarbonsäurederivaten unter Verwendung der N-Cyclopropylaniline.

EP 0 332 033 A2

## N-Cyclopropylaniline und deren Verwendung in Verfahren zur Herstellung von 1-Cyclopropyl-chinoloncarbonsäuren und deren Derivaten

Die Erfindung betrifft N-Cyclopropylaniline, die sich zur Herstellung von 4-Chinolon-3-carbonsäuren eignen, sowie Verfahren zur Herstellung von Chinoloncarbonsäurederivaten unter Verwendung der N-Cyclopropylaniline.

In 7-Stellung aminsubstituierte 4-Chinolon-3-carbonsäuren sind für die gute antibakterielle Wirkung bekannt. Aus dieser Gruppe ragen insbesondere die 1-Cyclopropyl-Verbindungen aufgrund ihres hohen Wirkniveaus heraus.

Beispielhaft für diese Klasse sei die 1-Cyclopropyl-6-fluor-1,4-dihydro-7-(1-piperazinyl)-4-oxo-3-chinolincarbonsäure (Ciprofloxacin) erwähnt.

Das erfindungsgemäße Verfahren erlaubt es nun, die aufgrund der exzellenten Wirkung wertvollen 1-Cyclopropylchinoloncarbonsäuren beziehungsweise deren Derivate herzustellen.

Die Erfindung betrifft ein Verfahren zur Herstellung von Chinoloncarbonsäurederivaten der Formel (I)

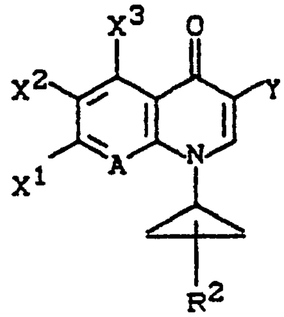

(I)

in der

$R^2$ für Wasserstoff, $C_1$-$C_3$-Alkyl oder Halogen steht,

$X^1$ Wasserstoff, $C_1$-$C_3$-Alkyl, Halogen oder $NR^8R^9$ bedeutet, wobei

$R^8$ und $R^9$ gemeinsam mit dem Stickstoffatom, an das sie gebunden sind einen 5- oder 6-gliedrigen Ring bilden, der als Ringglieder zusätzlich die Atome bzw. Gruppen $>NR^{10}$ oder O enthalten kann und der gegebenenalls ein- bis zweifach durch $C_1$-$C_3$-Alkyl, Hydroxy, $C_1$-$C_3$-Alkoxy, insbesondere Methoxy oder $NR^{11}R^{12}$ substituiert sein kann, wobei

$R^{10}$ und $R^{11}$ gleich oder verschieden sein können und für Wasserstoff oder $C_1$-$C_3$-Alkyl stehen und

$R^{12}$ Wasserstoff, $C_1$-$C_3$-Alkyl, Acetyl oder t-Butoxycarbonyl bedeutet,

$X^2$ und $X^3$ gleich oder verschieden sein können und für Wasserstoff, Nitro oder Halogen stehen,

A für Stickstoff oder einen Rest C-$X^4$ steht, wobei

$X^4$ für Wasserstoff, $C_1$-$C_3$-Alkyl, Alkoxy mit 1-3 Kohlenstoffatomen, Alkylmercapto mit 1-3 Kohlenstoffatomen, Halogen, Nitro, Cyano oder Alkoxycarbonyl mit bis zu 3 Kohlenstoffatomen in Alkoholteil steht und

Y eine Nitrilgruppe, eine Estergruppe $COOR^5$ oder eine Säureamidgruppe $CONR^6R^7$ darstellt, wobei $R^5$ für $C_1$-$C_3$-Alkyl steht und $R^6$ und $R^7$ gleich oder verschieden sein können und für Wasserstoff oder $C_1$-$C_3$-Alkyl stehen und $R^6$ gegebenenfalls substituiertes Phenyl sein kann,

dadurch gekennzeichnet, daß man in einem ersten Schritt Aniline der Formel (II)

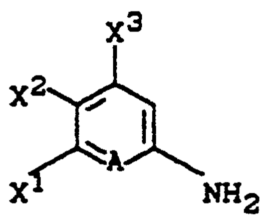

(II)

in der $X^1$, $X^2$, $X^3$ und A die oben angegebene Bedeutung haben mit Cyclopropanderivaten der Formel (III)

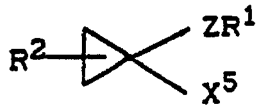

(III)

in der
$X^5$ für Halogen steht,
$R^1$ Alkyl mit 1-3 Kohlenstoffatomen bedeutet,
$R^2$ die oben angegebene Bedeutung hat und
Z Sauerstoff oder Schwefel darstellt
unter $HX^5$-Abspaltung zu N-Cyclopropylanilinen (IV) umsetzt,

(II) + (III)

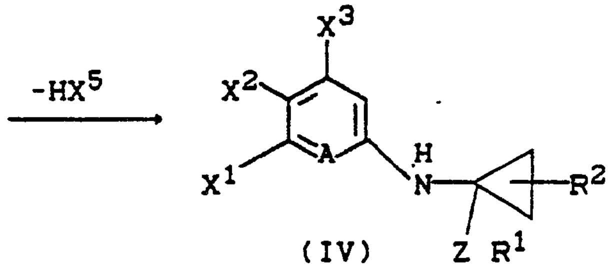


(IV)

die durch Reduktion in Verbindungen der Formel (V)

(IV)

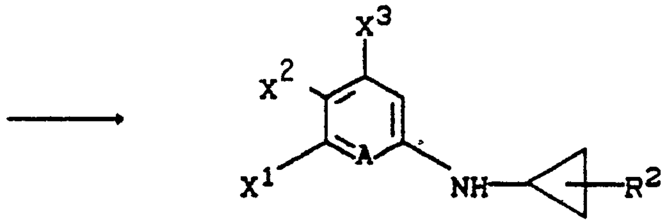

(V)

überführt werden,
welche man anschließend mit einem Alkenderivat der Formel (VI)

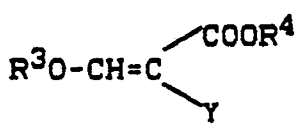

(VI)

in der
Y die oben angegebene Bedeutung hat und
$R^3$ und $R^4$ gleich oder verschieden sein können und für $C_1$-$C_3$-Alkyl stehen,
unter Abspaltung von $R^3OH$ und $R^4OH$ nach folgendem Schema zum Chinoloncarbonsäurederivat (I) umsetzt.

$$(V) + (VI) \xrightarrow[-R^4OH]{-R^3OH} (I)$$

Die Verbindungen, in denen $R^{11}$ und $R^{12}$ Wasserstoff bzw. $R^{11}$ Wasserstoff und $R^{12}$ $C_1$-$C_3$-Alkyl bzw. vice versa bedeuten, werden gegebenenfalls durch Abspaltung der vorher eingebrachten Schutzgruppen Acetyl oder tert. -Butoxycarbonyl hergestellt.

Bevorzugt ist ein Verfahren zur Herstellung von Chinoloncarbonsäurederivaten der Formel (I)

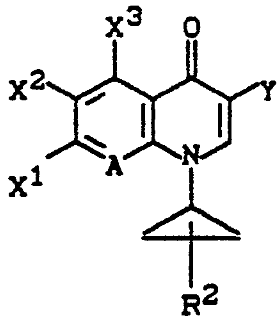

(I)

in der
$R^2$ für Wasserstoff oder Halogen steht,
$X^1$ Halogen oder $NR^8R^9$ bedeutet, wobei
$R^8$ und $R^9$ gemeinsam mit dem Stickstoffatom, an das sie gebunden sind einen 5- oder 6-gliedrigen Ring bilden, der als Ringglieder zusätzlich die Atome bzw. Gruppen $>NR^{10}$ oder O enthalten kann und der gegebenenfalls ein- bis zweifach durch $C_1$-$C_3$-Alkyl, Hydroxy, Methoxy oder $NR^{11}$-$R^{12}$ substituiert sein kann, wobei
$R^{10}$ und $R^{11}$ gleich oder verschieden sein können und für Wasserstoff oder $C_1$-$C_3$-Alkyl stehen und
$R^{12}$ Wasserstoff, $C_1$-$C_3$-Alkyl, Acetyl oder t-Butoxycarbonyl bedeutet,
$X^2$ für Fluor steht
$X^3$ Wasserstoff, Nitro oder Halogen bedeutet,
A für Stickstoff oder einen Rest C-$X^4$ steht, wobei
$X^4$ für Wasserstoff, Halogen oder Nitro steht und
Y eine Nitrilgruppe oder eine Estergruppe $COOR^5$ darstellt, wobei
$R^5$ für $C_1$-$C_3$-Alkyl steht,
das dadurch gekennzeichnet ist, daß man in einem ersten Schritt Aniline der Formel (II)

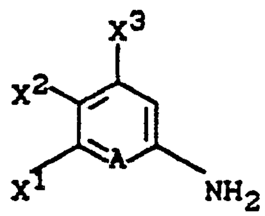

(II)

in der $X^1$, $X^2$, $X^3$ und A die oben angegebene Bedeutung haben mit Cyclopropanderivaten der Formel (III),

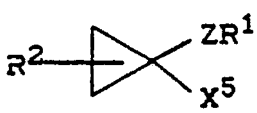

(III)

in der
$X^5$ für Chlor oder Brom steht,
$R^1$ Alkyl mit 1-3 Kohlenstoffatomen bedeutet,
$R^2$ die oben angegebene Bedeutung hat und
Z Sauerstoff oder Schwefel darstellt
unter $HX^5$-Abspaltung nach folgendem Schema zu N-Cyclopropylanilinen (IV) umsetzt,

(II) + (III)

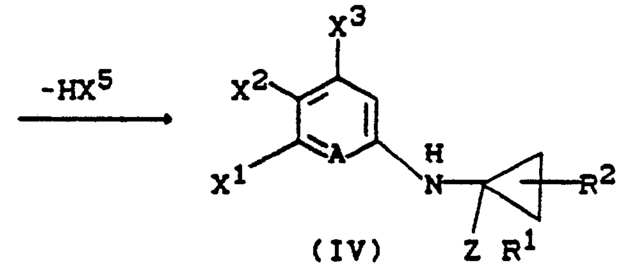


(IV)

die durch Reduktion in Verbindungen der Formel (V)

(IV)

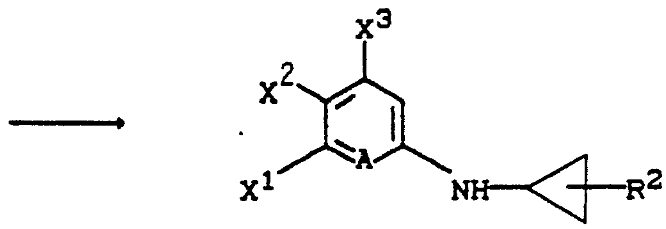

(V)

überführt werden, welche man anschließend mit einem Alkenderivat der Formel (VI)

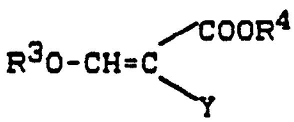

(VI)

in der
Y die oben angegebene Bedeutung hat und
$R^3$ und $R^4$ gleich oder verschieden sein können und für Methyl oder Ethyl stehen,
unter Abspaltung von $R^3OH$ und $R^4OH$ nach folgendem Schema zum Chinoloncarbonsäurederivat (I) umsetzt.

$$(V) + (VI) \xrightarrow[-R^4OH]{-R^3OH} (I)$$

Besonders bevorzugt ist ein Verfahren zur Herstellung von Chinoloncarbonsäurederivaten der Formel (I)

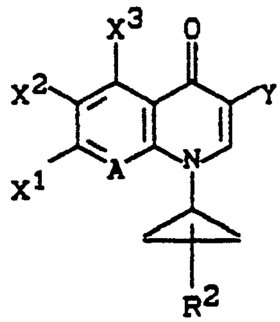

(I)

in der
$R^2$ für Fluor oder Chlor steht, ganz besonders aber Wasserstoff bedeutet,
$X^1$ Chlor, Fluor oder $NR^8R^9$ bedeutet, wobei

$R^8$ und $R^9$ gemeinsam mit dem Stickstoffatom, an das sie gebunden sind einen 5- oder 6-gliedrigen Ring bilden, der als Ringglied zusätzlich die Gruppe $>NR^{10}$ enthalten kann und der gegebenenfalls ein-bis zweifach durch Methyl, Hydroxy, Methoxy oder $NR^{11}R^{12}$ substituiert sein kann, wobei
$R^{10}$ und $R^{11}$ gleich oder verschieden sein können und für Wasserstoff oder Methyl stehen und
$R^{12}$ Wasserstoff, $C_1$-$C_3$-Alkyl, Acetyl oder t-Butoxycarbonyl bedeutet,
$X^2$ für Fluor steht,
$X^3$ Wasserstoff oder Nitro bedeutet,
A für C-$X^4$ steht, wobei
$X^4$ für Wasserstoff, Methyl, Fluor oder Chlor steht und
Y die Estergruppe $COOR^5$ darstellt, wobei
$R^5$ für Methyl oder Ethyl steht,
das dadurch gekennzeichnet ist, daß man in einem ersten Schritt Aniline der Formel (II)

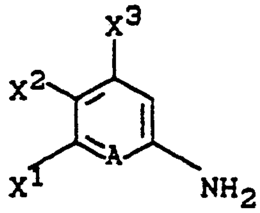

(II)

in der $X^1$, $X^2$, $X^3$ und A die oben angegebene Bedeutung haben mit Cyclopropanderivaten der Formel (III)

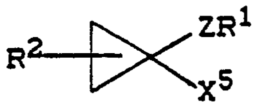

(III)

in der
$X^5$ für Brom steht,
$R^1$ Ethyl bedeutet,
$R^2$ die oben angegebene Bedeutung hat und
Z Sauerstoff oder Schwefel bedeutet
unter $HX^5$-Abspaltung zu N-Cyclopropylanilinen (IV) umsetzt,

(II) + (III)

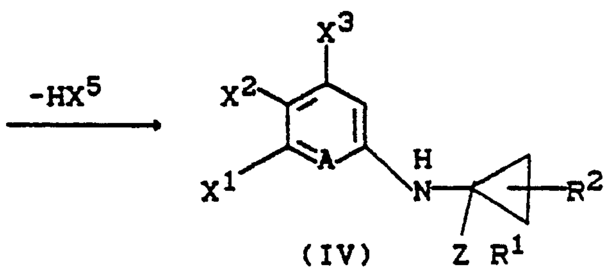


(IV)

die durch Reduktion in Verbindungen der Formel (V)

(IV)

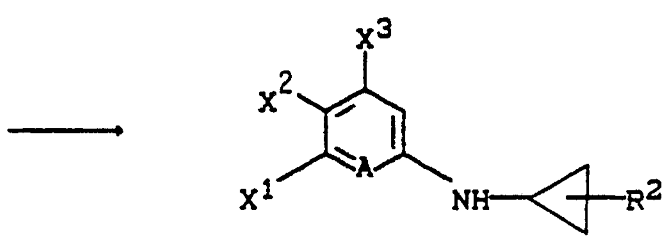

(V)

überführt werden, welche man anschließend mit einem Alkenderivat der Formel (VI)

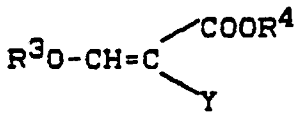

(VI)

in der

Y die oben angegebene Bedeutung hat und

$R^3$ für Methyl steht und

$R^4$ Methyl oder Ethyl bedeutet

unter Abspaltung von $R^3OH$ und $R^4OH$ nach folgendem Schema zum Chinoloncarbonsäurederivat (I) umsetzt.

$$(V) + (VI) \xrightarrow[-R^4OH]{-R^3OH} (I)$$

Die Chinoloncarbonsäurederivate (I) können auf bekannte Weise sauer oder alkalisch in die antibakteriell wirksamen Chinoloncarbonsäuren umgewandelt werden.

Verwendet man beispielsweise 3,4-Difluoranilin und 1-Brom-1-ethoxycyclopropan als Ausgangsmaterialien im ersten Schritt, so erhält man N-(1-Ethoxycyclopropyl)-3,4-difluoranilin

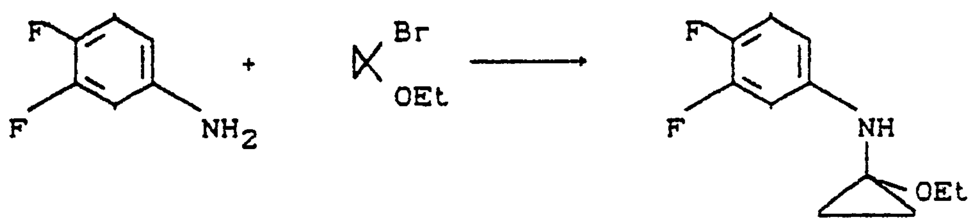

(1) (2) (3)

Nach der reduktiven Entfernung von -OEt und der Umsetzung mit Methoxymethylenmalonester (5) wird der bekannte 1-Cyclopropyl-6,7-difluor-1,4-dihydro-4-oxochinolincarbonsäureethylester (6) erhalten.

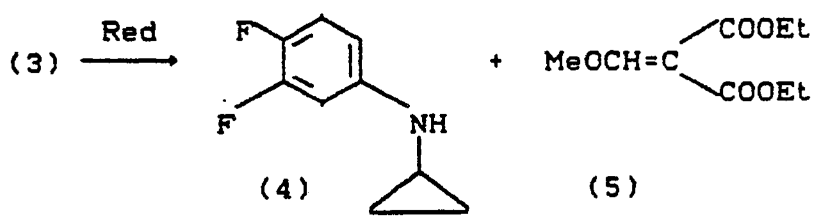


(3) (4) (5)

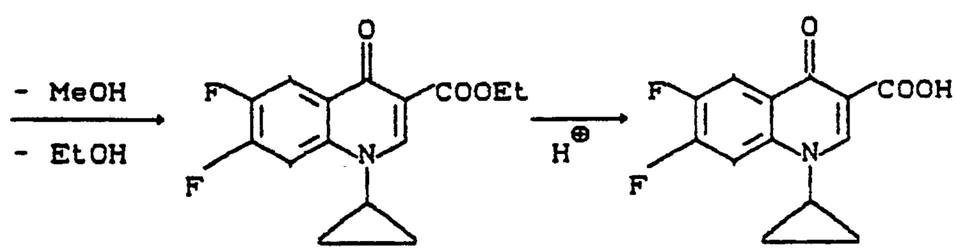


(6) (7)

Die gemäß diesem Verfahren als Ausgangsstoffe benötigten Aniline II sind bekannt oder können nach bekannten Methoden hergestellt werden.

Als Beispiele seien genannt:

3-Chlor-4-fluoranilin, 3,4-Difluoranilin, 2,3,4-Trifluoranilin, 3-Chlor-4-nitroanilin, 3,4-Difluor-2-methylanilin, 3,4-Dichlor-2-methylanilin, 3,4-Methylendioxy-anilin, 2,3,4,5-Tetrafluoranilin, 2-Chlor-3,4-difluoranilin, 2-Brom-3,4-difluoranilin, 2-Aminopyridin, 2-Amino-6-methylpyridin, 2-Amino-5-chlorpyridin, 2-Amino-5-brom-pyridin.

Die als Ausgangsstoffe benötigten Cyclopropanderivate der Formel (III) sind bekannt (J. Org. Chem. 1985, 3255; Recl. Trav. Chim. Pays-Bas 100, 184).

Als Beispiele seien genannt:

1-Chlor-1-methoxycyclopropan, 1-Brom-1-methoxycyclopropan, 1-Brom-1-ethoxycyclopropan, 1-Brom-1-methylmercapto-cyclopropan, 1-Chlor-1-methylmercaptocyclopropan.

Weitere Ausgangsstoffe werden in Recl. Trav. Chim. Pays-Bas, 100, 184 (1981) z.B.

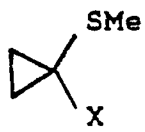

(X = Cl, Br)

beschrieben.

Die als Ausgangsstoffe benötigten Alkenderivate der Formel (V) sind bekannt.

Als Beispiele seinen genannt:

Methoxymethylenmalonsäurediethylester, Methoxymethylenmalonsäuredimethylester, Methoxymethylencyanessigsäureethylester.

Die Substitutionsreaktionen (II) + (III) → (IV) werden in einem Temperaturbereich von 20-120 °C, bevorzugt 40-80 °C durchgeführt.

Als Verdünnungsmittel für diese Reaktion können verwendet werden: Pentan, Hexan, Heptan, Benzol, Toluol, Tetrachlorkohlenstoff, Chloroform, Methylenchlorid oder Gemische dieser Lösungsmittel.

Als Säurefänger für diese Reaktion können eingesetzt werden:

Triethylamin, Diazabicyclo[2,2,2]octan, 1,8-Diazabicyclo[5.4.0]undec-7-en, Pyridin, Kaliumcarbonat, Natriumhydrogencarbonat, Dimethylbenzylamin.

Als Reduktionsmittel für den Reduktionsschritt (IV) → (V) finden Verwendung: Natriumcyanoborhydrid, Natriumborhydrid, Lithiumaluminiumhydrid und bevorzugt ein 1:1-Gemisch aus Natriumborhydrid und Bortrifluorid-Diethylether-Komplex.

Diese Reaktion kann in einem Verdünnungsmittel wie z.B. Dioxan oder Tetrahydrofuran durchgeführt werden.

Die Reaktionen (V) + (VI) → (I) werden in einem Temperaturbereich von 50-300 °C, bevorzugt 100-250 °C durchgeführt.

Die Abspaltung von $R^3OH$ kann durch Erhitzen der reinen Komponenten oder aber unter Zusatz eines Verdünnungsmittels wie zum Beispiel Diphenylether, Biphenyl oder eines Gemisches der beiden Verdünnungsmittel erfolgen.

Die Cyclisierung zu (I) (Abspaltung von $R^4OH$) wird unter Zusatz eines Verdünnungsmittels wie zum Beispiel Diphenylether, Biphenyl oder Polyphosphorsäure durchgeführt.

Die Reaktion von (V) mit (VI) kann durch Zusatz von Basen wie Diazabicyclo[5.3.0]undec-7-en, 1,4-Diazabicyclo[2.2.2]octan, Natriumhydrid oder Kaliumtertiärbutylat durchgeführt werden.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Folgende Beispiele erläutern die Erfindung:

## Beispiel 1

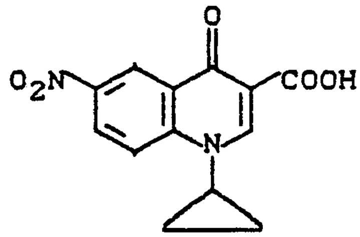

Eine Mischung aus 2,9 g (20 mmol) 4-Nitroanilin, 4,95 g (30 mmol) 1-Brom-1-ethoxy-cyclopropan und 2 g (30 mmol) Triethylamin in 15 ml Dichlormethan wird 3 Tage unter Rückfluß erhitzt. Man schüttelt mit Wasser aus, trocknet mit Natriumsulfat, engt ein und reinigt den Rückstand durch Chromatographie an Kieselgel mit Dichlormethan als Laufmittel. Zur Reduktion stellt man eine Mischung aus 1,1 g (30 mmol) Natriumborhydrid und 4 g (30 mmol) Borfluorid-etherat in 30 ml absolutem Tetrahydrofuran unter Wasserausschluß und Eiskühlung her, rührt 1 Stunde unter Eiskühlung, fügt das erhaltene 4-(1-Ethoxycyclopropylamino)-nitrobenzol (3,2 g) zu und erhitzt 1,5 Stunden unter Rückfluß. Das Reaktionsgemisch wird eingeengt, in 30 ml Dichlormethan aufgenommen, mit Wasser gewaschen, mit Natriumsulfat getrocknet, eingeengt und aus Ethanol umkristallisiert. Das erhaltene 4-Cyclopropylamino-nitrobenzol (2,1 g, Schmelzpunkt 134-136 °C) wird in 10 ml Dimethylformamid mit 3,3 g (15 mmol) Ethoxymethylenmalonsäurediethylester versetzt und unter Eiskühlung innerhalb 30 Minuten 375 mg 80 %iges Natriumhydrid unter Wasserausschluß zugefügt. Man läßt 30 Minuten unter Eiskühlung und 1 Stunde bei Raumtemperatur rühren und gießt auf eine Mischung aus 25 ml Eiswasser und 1,4 ml Essigsäure. Der ausgefallene N-(4-Nitrophenyl)-N-cyclopropyl-amino-methylen-malonsäurediethylester wird abgesaugt, mit Wasser und Methanol gewaschen und getrocknet (2,3 g, Schmelzpunkt 116-120 ° [aus Isopropanol umkristallisiert]). Dieses Produkt wird mit 9 g Polyphosphorsäure 1 Stunde auf 145 °C erhitzt, die Mischung abgekühlt und in 50 ml Eiswasser eingetragen. Der angefallene dunkle Niederschlag wird abgesaugt und durch Verrühren mit Dichlormethan/Methanol (95 : 5) und Umkristallisation aus Glykolmonomethylether gereinigt: 1,2 g 1-Cyclopropyl-1,4-dihydro-6-nitro-4-oxo-3-chinolincarbonsäureethylester; Schmelzpunkt 226 - 228 °C (unter Zersetzung).

Der aus diese Weise erhaltene Ester wird in einer Mischung aus 7,6 ml Essigsäure, 5,4 ml Wasser und 0,9 ml Schwefelsäure 1 Stunde unter Rückfluß erhitzt und die Suspension im 40 ml Eiswasser eingegossen. Der ausgefallene Niederschlag wird abgesaugt, mit Wasser gewaschen und getrocknet: 0,95 g 1-Cyclopropyl-1,4-dihydro-6-nitro-4-oxo-3-chinolincarbonsäure; Schmelzpunkt 256 - 262 °C (unter Zersetzung).

Beispiel 2

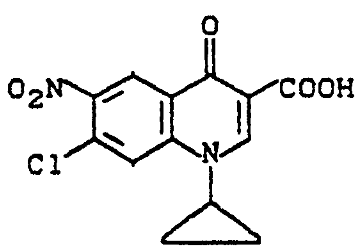

Analog Beispiel 1 wird 3-Chlor-4-nitro-anilin über 4-Cyclopropylamino-2-chlor-nitrobenzol (Schmelzpunkt 94 -98 °C) zu 7-Chlor-1-cyclopropyl-1,4-dihydro-6-nitro-4-oxo-3-chinolincarbonsäure vom Schmelzpunkt 278 - 281 °C (unter Zersetzung) umgesetzt.

Beispiel 3

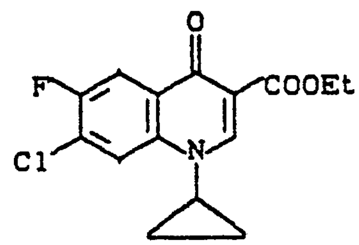

4,37 g 3-Chlor-4-fluoranilin (30 mmol), 7,4 g 1-Brom-1-ethoxy-cyclopropan (45 mmol) und 4,5 g Triethylamin (45 mmol) werden 48 Stunden in 30 ml Methylenchlorid gekocht. Danach wird mehrfach mit Wasser gewaschen, über Natriumsulfat getrocknet und eingeengt. Der Rückstand wird durch Chromatographie an Kieselgel mit Methylenchlorid als Laufmittel gereinigt.

Zur Reduktion stellt man eine Mischung aus 2,28 g Natriumborhydrid (60 mmol) und 8,5 g Bortrifluoridetherat (60 mmol) in 60 ml absolutem Tetrahydrofuran unter Eiskühlung her, rührt eine Stunde unter Eiskühlung und fügt das erhaltene 3-Chlor-N-(1-ethoxycyclopropyl)-4-fluoranilin zu und erhitzt 1,5 Stunden unter Rückfluß. Danach engt man das Reaktionsgemisch ein, nimmt in Methylenchlorid auf, wäscht mit Wasser, trocknet über Natriumsulfat und engt ein.

Das erhaltene Rohprodukt (5,4 g) wird mit 6,1 g Methoxymethylenmalonsäurediethylester in 50 ml Diphenylether 30 Minuten auf 240 °C erhitzt. Danach wird das Lösungsmittel im Vakuum entfernt. Man kristallisiert den Rückstand aus Glykolmonomethylether um und erhält 5,6 g 7-Chlor-1-cyclopropyl-6-fluor-1,4-dihydro-4-oxo-3-chinolincarbonsäureethylester.

Schmelzpunkt 219 - 221 °C

Lit (Liebigs Ann. Chem. 1987, 29): 220 °C

Beispiel 4

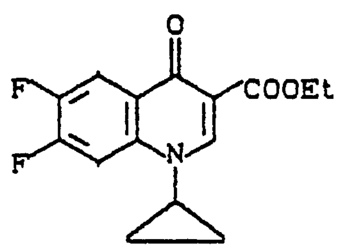

Analog Beispiel 3 erhält man ausgehend von 3,4-Difluoranilin 1-Cyclopropyl-6,7-difluor-1,4-dihydro-4-oxo-3-chinolincarbonsäureethylester.

Schmelzpunkt 225 - 227 °C.

Beispiel 5

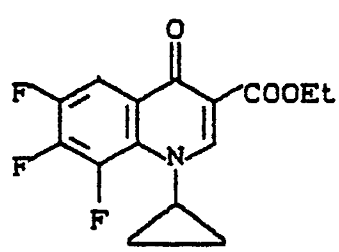

Analog Beispiel 3 erhält man ausgehend von 2,3,4-Trifluoranilin 1-Cyclopropyl-6,7,8-trifluor-1,4-dihydro-4-oxo-3-chinolincarbonsäureethylester.

Schmelzpunkt 167 - 168 °C.

**Ansprüche**

1. Verfahren zur Herstellung von Chinoloncarbonsäurederivaten der Formel (I)

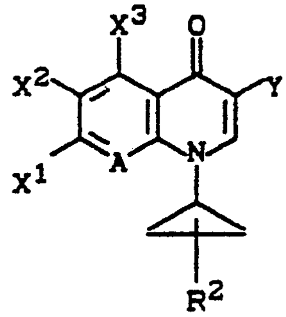

(I)

in der
$R^2$ für Wasserstoff, $C_1$-$C_3$-Alkyl oder Halogen steht,
$X^1$ Wasserstoff, $C_1$-$C_3$-Alkyl, Halogen oder $NR^8R^9$ bedeutet, wobei
$R^8$ und $R^9$ gemeinsam mit dem Stickstoffatom, an das sie gebunden sind einen 5- oder 6-gliedrigen Ring bilden, der als Ringglieder zusätzlich die Atome bzw. Gruppen $>NR^{10}$ oder O enthalten kann und der gegebenenfalls ein- bis zweifach durch $C_1$-$C_3$-Alkyl, Hydroxy, $C_1$-$C_3$-Alkoxy oder $NR^{11}R^{12}$ substituiert sein kann, wobei
$R^{10}$ und $R^{11}$ gleich oder verschieden sein können und für Wasserstoff oder $C_1$-$C_3$-Alkyl stehen und
$R^{12}$ $C_1$-$C_3$-Alkyl, Wasserstoff, Acetyl oder t-Butoxycarbonyl bedeutet,
$X^2$ und $X^3$ gleich oder verschieden sein können und für Wasserstoff, Nitro oder Halogen stehen,
A für Stickstoff oder einen Rest C-$X^4$ steht, wobei
$X^4$ für Wasserstoff, $C_1$-$C_3$-Alkyl, Alkoxy mit 1-3 Kohlenstoffatomen, Alkylmercapto mit 1-3 Kohlenstoffatomen, Halogen, Nitro, Cyano oder Alkoxycarbonyl mit bis zu 3 Kohlenstoffatomen im Alkoholteil steht und
Y eine Nitrilgruppe, eine Estergruppe $COOR^5$ oder eine Säureamidgruppe $CONR^6R^7$ darstellt, wobei $R^5$ für $C_1$-$C_3$-Alkyl steht und $R^6$ und $R^7$ gleich oder verschieden sein können und für Wasserstoff oder $C_1$-$C_3$-Alkyl stehen und $R^6$ gegebenenfalls substituiertes Phenyl sein kann,
dadurch gekennzeichnet, daß man in einem ersten Schritt Aniline der Formel (II)

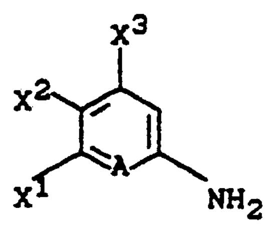

(II)

in der $X^1$, $X^2$, $X^3$ und A die oben angegebene Bedeutung haben mit Cyclopropanderivaten der Formel (III)

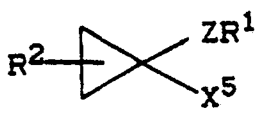

(III)

in der
$X^5$ für Halogen steht,
$R^1$ Alkyl mit 1-3 Kohlenstoffatomen bedeutet,
$R^2$ die oben angegebene Bedeutung hat und
Z Sauerstoff oder Schwefel darstellt
unter $HX^5$-Abspaltung zu N-Cyclopropylanilinen (IV) nach dem folgenden Schema umsetzt

(II) + (III)

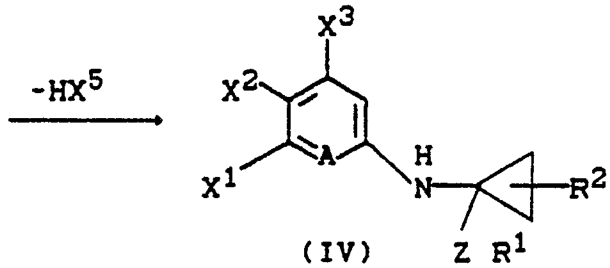


(IV)

die durch Reduktion in Verbindungen der Formel (V)

(IV)

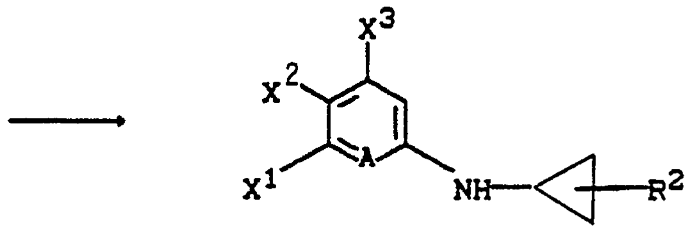

(V)

überführt werden,
welche man anschließend mit einem Alkenderivat der Formel (VI)

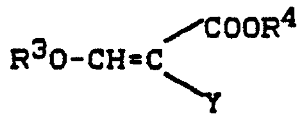

(VI)

in der
Y die oben angegebene Bedeutung hat und
$R^3$ und $R^4$ gleich oder verschieden sein können und für $C_1$-$C_3$-Alkyl stehen,
unter Abspaltung von $R^3OH$ und $R^4OH$ zum Chinoloncarbonsäurederivat (I) umsetzt.

2. Verfahren nach Anspruch 1 zur Herstellung von Chinoloncarbonsäurederivaten der Formel (I)

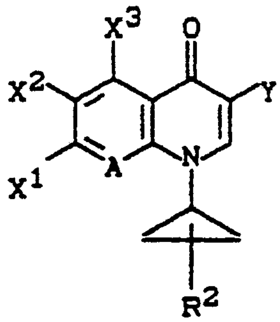

(I)

in der
$R^2$ für Wasserstoff oder Halogen steht,
$X^1$ Halogen oder $NR^8R^9$ bedeutet, wobei
$R^8$ und $R^9$ gemeinsam mit dem Stickstoffatomen, an das sie gebunden sind einen 5- oder 6-gliedrigen Ring bilden, der als Ringglieder zusätzlich die Atome bzw. Gruppen $>NR^{10}$ oder O enthalten kann und der gegebenenfalls ein- bis zweifach durch $C_1$-$C_3$-Alkyl, Hydroxy, Methoxy oder $NR^{11}R^{12}$ substituiert sein kann, wobei
$R^{10}$ und $R^{11}$ gleich oder verschieden sein können und für Wasserstoff oder $C_1$-$C_3$-Alkyl stehen und
$R^{12}$ Wasserstoff, $C_1$-$C_3$-Alkyl, Acetyl oder t-Butoxycarbonyl bedeutet,
$X^2$ für Fluor steht,
$Y^3$ Wasserstoff, Nitro oder Halogen bedeutet

A für Stickstoff oder einen Rest C-$X^4$ steht, wobei
$X^4$ für Wasserstoff, Halogen, Nitro steht und
Y eine Nitrilgruppe oder eine Estergruppe $COOR^5$ darstellt, wobei
$R^5$ für $C_1$-$C_3$-Alkyl steht,
dadurch gekennzeichnet, daß man in einem ersten Schritt Aniline der Formel (II)

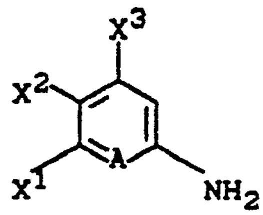

(II)

in der $X^1$, $X^2$, $X^3$ und A die oben angegebene Bedeutung haben mit Cyclopropanderivaten der Formel (III)

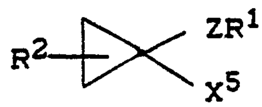

(III)

in der
$X^5$ für Chlor oder Brom steht,
$R^1$ Alkyl mit 1-3 Kohlenstoffatomen bedeutet,
$R^2$ die oben angegebene Bedeutung hat und
Z Sauerstoff oder Schwefel bedeutet
unter $HX^5$-Abspaltung nach folgendem Schema zu N-Cyclopropylanilinen (IV) umsetzt,

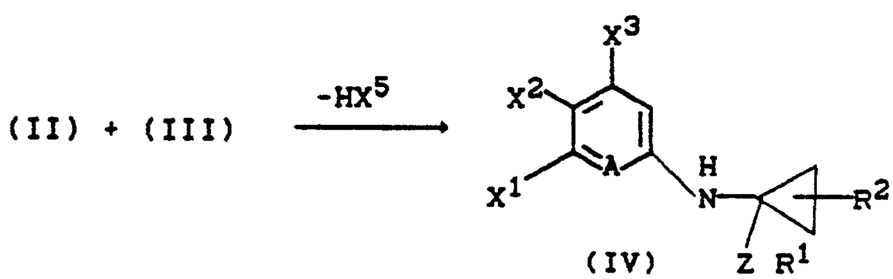

die durch Reduktion in Verbindungen der Formel (V)

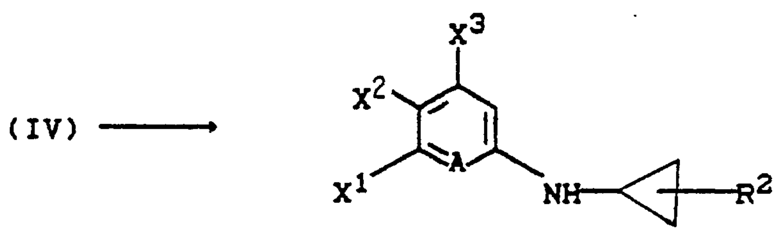

(V)

überführt werden,
welche man anschließend mit einem Alkenderivat der Formel (VI)

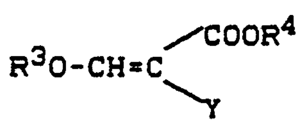

(VI)

in der
Y die oben angegebene Bedeutung hat und
$R^3$ und $R^4$ gleich oder verschieden sein können und für Methyl oder Ethyl stehen
unter Abspaltung von $R^3OH$ und $R^4OH$ zum Chinolonsäurederivat (I) umsetzt.

3. Verfahren nach Anspruch 1 zur Herstellung von Chinoloncarbonsäurederivaten der Formel (I)

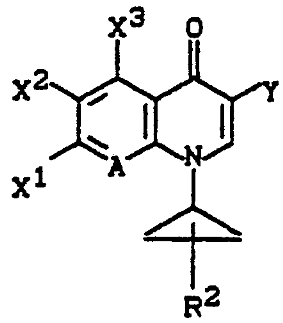

(I)

in der
$R^2$ für Fluor oder Chlor steht, ganz besonders bevorzugt aber Wasserstoff darstellt,
$X^1$ Chlor, Fluor oder $NR^8R^9$ bedeutet, wobei
$R^8$ und $R^9$ gemeinsam mit dem Stickstoffatom, an das sie gebunden sind einen 5- oder 6-gliedrigen Ring bilden, der als Ringglieder zusätzlich die Gruppe $>NR^{10}$ enthalten kann und der gegebenenfalls ein-bis zweifach durch Methyl, Hydroxy, Methoxy oder $NR^{11}R^{12}$ substituiert sein kann, wobei
$R^{10}$ und $R^{11}$ gleich oder verschieden sein können und für Wasserstoff oder Methyl stehen und
$R^{12}$ Wasserstoff, $C_1$-$C_3$-Alkyl, Acetyl oder t-Butoxycarbonyl bedeutet,
$X^2$ für Fluor steht,
$X^3$ Wasserstoff oder Nitro bedeutet,
A für C-$X^4$ steht, wobei
$X^4$ für Wasserstoff, Methyl, Fluor oder Chlor steht und
Y eine Estergruppe $COOR^5$ darstellt, wobei
$R^5$ für Methyl oder Ethyl steht,
dadurch gekennzeichnet, daß man in einem ersten Schritt Aniline der Formel (II)

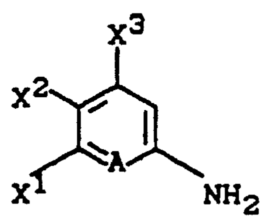

(II)

in der $X^1$, $X^2$, $X^3$ und A die oben angegebene Bedeutung haben mit Cyclopropanderivaten der Formel (III)

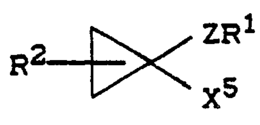

(III)

in der
$X^5$ für Brom steht,
$R^1$ Ethyl bedeutet,
$R^2$ die oben angegebene Bedeutung hat und
Z Sauerstoff oder Schwefel bedeutet
unter $HX^5$-Abspaltung nach folgendem Schema zu N-Cyclopropylanilinen (IV) umsetzt,

(II) + (III)

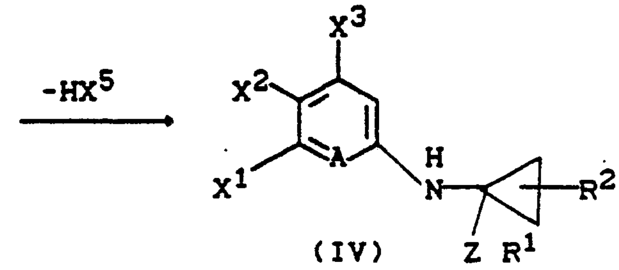


(IV)

die durch Reduktion in Verbindungen der Formel (V)

(IV)

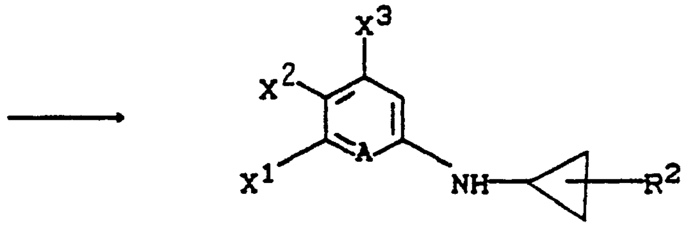

(V)

überführt werden,
welche man anschließend mit einem Alkenderivat der Formel (VI)

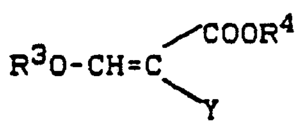

(VI)

in der
Y die oben angegebene Bedeutung hat und
$R^3$ für Methyl steht und
$R^4$ Methyl oder Ethyl bedeutet,
unter Abspaltung von $R^3OH$ und $R^4OH$ zum Chinoloncarbonsäurederivat (I) umsetzt.

4. N-Cyclopropylaniline der Formel (IV)

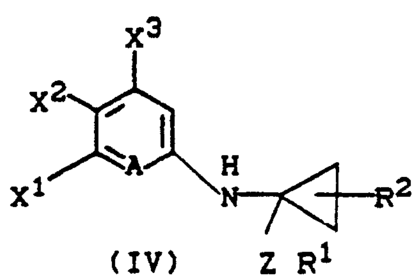

(IV)

(IV)

in der
$R^2$ für Wasserstoff, $C_1$-$C_3$-Alkyl oder Halogen steht,
$X^1$ Wasserstoff, $C_1$-$C_3$-Alkyl, Halogen oder $NR^8R^9$ bedeutet, wobei
$R^8$ und $R^9$ gemeinsam mit dem Stickstoffatom, an das sie gebunden sind, einen 5- oder 6-gliedrigen Ring bilden, der als Ringglieder zusätzlich die Atome bzw. Gruppen $>NR^{10}$ oder O enthalten kann und der gegebenenfalls ein- bis zweifach durch $C_1$-$C_3$-Alkyl, Hydroxy, Methoxy oder $NR^{11}R^{12}$ substituiert sein kann, wobei
$R^{10}$ und $R^{11}$ gleich oder verschieden sein können und für Wasserstoff oder $C_1$-$C_3$-Alkyl stehen und
$R^{12}$ Wasserstoff, $C_1$-$C_3$, Acetyl oder t-Butoxycarbonyl bedeutet
$X^2$ und $X^3$ gleich oder verschieden sein können und für Wasserstoff, Nitro oder Halogen stehen,

A für Stickstoff oder einen Rest C-$X^4$ steht, wobei
$X^4$ für Wasserstoff, $C_1$-$C_3$-Alkyl, Alkoxy mit 1-3 Kohlenstoffatomen, Alkylmercapto mit 1-3 Kohlenstoffatomen, Halogen, Nitro, Cyano oder Alkoxycarbonyl mit bis zu 3 Kohlenstoffatomen im Alkoholteil steht und
$R^1$ Alkyl mit 1-3 Kohlenstoffatomen bedeutet und
Z Sauerstoff oder Schwefel darstellt.

5. Verwendung von N-Cyclopropylanilinen der Formel (IV) nach Anspruch 4 zur Herstellung von Chinoloncarbonsäurederivaten.